# EUROPÄISCHE PATENTSCHRIFT

(11) **EP 4 537 884 B1**
(45) Veröffentlichungstag und Bekanntmachung des Hinweises auf die Patenterteilung: **25.03.2026**
(21) Anmeldenummer: 24203911.3
(22) Anmeldetag: 01.10.2024
(51) Int. Cl.: A61M 16/08

(54) **VORRICHTUNG UND SYSTEM ZUR ERZEUGUNG EINES KONTINUIERLICHEN POSITIVEN ATEMWEGSDRUCKS, INSBESONDERE ZUR ATMUNGSUNTERSTÜTZUNG BEI FRÜH- UND NEUGEBORENEN**
DEVICE AND SYSTEM FOR GENERATING CONTINUOUS POSITIVE AIRWAY PRESSURE, IN PARTICULAR FOR RESPIRATORY ASSISTANCE IN PREMATURE INFANTS AND NEONATES
DISPOSITIF ET SYSTÈME DE GÉNÉRATION D'UNE PRESSION POSITIVE CONTINUE DES VOIES AÉRIENNES, EN PARTICULIER POUR L'ASSISTANCE RESPIRATOIRE CHEZ LES NOUVEAU-NÉS ET PRÉMATURÉS

(30) Priorität: 09.10.2023 IT 202300020877
(43) Veröffentlichungstag der Anmeldung: 16.04.2025
(73) Patentinhaber: Pfm medical GmbH, 50996 Köln (DE)
(72) Erfinder: Welfers, Pia, 41844 Wegberg (DE); Haase, Bianca, 72070 Tübingen (DE); Kox, Georg, 41844 Wegberg (DE); Rauch, Daniel, 45147 Essen (DE)
(74) Vertreter: Lippert Stachow Patentanwälte Rechtsanwälte Part.mbB

(56) Entgegenhaltungen:
- WO-A1-99/24101
- US-A- 3 977 432
- US-A1- 2016 158 477

## Beschreibung

Die Erfindung betrifft eine Vorrichtung und ein System zur Erzeugung eines kontinuierlichen positiven Atemwegsdrucks, auch bezeichnet als CPAP (Continuous Positive Airway Pressure) Vorrichtung. Die CPAP-Vorrichtung ist beispielsweise als nasale CPAP-Vorrichtung (nCPAP) ausgebildet. CPAP-Vorrichtungen werden insbesondere auch zur Atmungsunterstützung von Früh- und Neugeborenen bei Frühgeburtlichkeitsassoziiertem Surfactantmangel oder transitorischer Tachypnoe des Neugeborenen eingesetzt.

Das Funktionsprinzip von CPAP-Vorrichtungen basiert darauf, dass der Patient gegen einen Druck atmet, der höher liegt als der Atmosphärendruck. Durch die Erzeugung eines PEEP (Positiver Endexpiratorischer Druck) in den Alveolen durch eine CPAP-Vorrichtung wird versucht, einen Kollaps dieser zu verhindern, um Atelektasen und somit einer Reduktion der funktionellen Residualkapazität vorzubeugen.

Im Gegensatz dazu stellen normale Beatmungsvorrichtungen beispielsweise nur während des Einatmens eine Luftzufuhr zur Verfügung und während des Ausatmens wird ein Unterdruck erzeugt. So offenbart beispielsweise die DE 31 19 814 A1 ein Beatmungsgerät mit einer steuerbaren Atemgasquelle und einer Vorrichtung zur Unterdruckerzeugung, welches den Patienten während der Inspirationsphase mit hochfrequenten Gashochdruckimpulsen versorgt. Das Beatmungsgerät speist über die steuerbare Atemgasquelle wenigstens eine Jet-Düse in einem Trachealtubus mit den Gashochdruckimpulsen. Zur Verbesserung der Wirksamkeit in der Exspirationsphase und insbesondere der CO₂-Elimination unter gleichzeitiger Vermeidung einer Behinderung spontaner Atemvorgänge, wie Hustenstöße, ist vorgesehen, dass der Trachealtubus wenigstens in der Exspirationsphase mit einer Vorrichtung zur Unterdruckerzeugung verbunden ist, welche in beiden Atemphasen eine Verbindung des proximalen Endes des Tubus mit der Umgebungsatmosphäre herstellt. Das Beatmungsgerät ist bei atmungsgestörten Patienten verwendbar, aber aufgrund des erzeugten Unterdrucks in der Exspirationsphase wird das Kollabieren der Alveolen bei Früh- und Neugeborenen nicht verhindert. Weitere vergleichbare Beatmungsvorrichtungen sind beispielsweise aus der US 3,977,432 A oder US 3,794,072 A bekannt.

Die EP 0 447 443 A1 offenbart eine Vorrichtung zur Erzeugung eines kontinuierlichen, positiven Atemwegdrucks (CPAP) mit Hilfe eines Ejektionsvorgangs, mit einem Atmungskanal, der sich an seinem freien Ende zur Atmosphäre hin öffnet und an seinem anderen Ende mit einem Kupplungsstück zum Anbringen an der Nase und/oder dem Mund des Patienten versehen werden kann, und mit einem Einlasskanal für Frischgas, der mit dem Atmungskanal an einer Stelle zwischen dessen Enden verbunden ist und dessen Durchfluss einstellbar ist, um einen einstellbaren positiven Druck im Atmungskanal zu erzielen. Der Atmungskanal weist einen ersten Abzweigkanal, der an dem Kupplungsstück anschließbar ist, und einen zweiten Abzweigkanal auf, der zur Atmosphäre hin offen ist, wobei die beiden Abzweigkanäle einen Winkel miteinander bilden. Der Einlasskanal liegt im Wesentlichen in der Verlängerung des ersten Abzweigkanals und ist so mit dem zweiten Abzweigkanal verbunden, dass der Frischgasstrom hauptsächlich koaxial in den ersten Abzweigkanal gerichtet ist und dadurch einen Ejektionsvorgang bewirkt. Die Querschnittsfläche des jeweiligen Abzweigkanals ist um ein Vielfaches größer als die kleinste Querschnittsfläche des Einlasskanals. Die Länge jedes Abzweigkanals ist relativ kurz, und beträgt vorzugsweise das Fünffache seines Innendurchmessers. Der Atmungskanal ist mit dem Einlasskanal zusammengebaut, um eine kompakte Einheit zu bilden, die an der Nase und/oder dem Mund des Patienten mittels eines Klebestreifens oder entsprechenden Mittels angebracht werden kann.

Die WO 99/24101 A1 beanstandet an den beiden vorgenannten CPAP- bzw. nCPAP-Vorrichtungen den komplizierten Aufbau, insbesondere die separaten Ein- und Auslasskanäle, wodurch die Vorrichtungen nicht einfach sterilisierbar sind, und teure Wegwerfartikel darstellen. Ferner seien die Vorrichtungen nicht flexibel in der Handhabung und in der Verbindung mit Druckluft-Erzeugungseinrichtungen und Druckmessgeräten. Gemäß der WO 99/24101 A1 werden diese Nachteile vermieden durch eine Vorrichtung zur Erzeugung eines kontinuierlichen positiven Atemwegdrucks (CPAP-Vorrichtung), insbesondere nasale CPAP-Vorrichtung (nCPAP-Vorrichtung), mit einem Hohlkörper, in dem ein Überdruck aufbaubar ist; einer in einer Seitenwand des Hohlkörpers vorgesehenen ersten Öffnung zum Zuführen einer in den Hohlkörper gerichteten Luftströmung und zum Abführen der ausgeatmeten Luftströmung; einem am Hohlkörper anbringbaren Anschlussstück zur Verbindung des Hohlkörpers mit einem Nasen- und/oder Mundansatzstück; und einem am Hohlkörper anbringbaren Abstandshalter, an dem eine Strömungsdüse zum Richten der Luftströmung auf die Öffnung anbringbar ist.

Die EP 1 897 577 A1 offenbart eine Weiterbildung der Vorrichtung aus der WO 99/24101 A1 mit einem zusätzlichen Einlass für eine Medikamentenströmung.

Die EP 0 658 356 A1 offenbart ferner eine CPAP-Vorrichtung mit einem Paar von Nasenansatzstücken jeweils mit einer Kanülenspitze zum Einsetzen in die Nasenlöcher eines Patienten.

Die GB 2570052 B offenbart eine CPAP-Vorrichtung mit mehreren verschließbaren Ports, um eine Vielzahl unterschiedlicher Beatmungstherapien bereitstellen zu können.

Die EP 2 269 676 A2 offenbart eine CPAP-Vorrichtung mit einem steuerbaren Ventil in der Druckluftzuführung, um den zugeführten Luftstrom anzupassen.

Insbesondere bei der Atmungsunterstützung bei Früh- und Neugeborenen mittels einer CPAP-Vorrichtung ist darauf zu achten, dass die Belastung auf den Kopf des Früh- und Neugeborenen so gering wie möglich ist, da die Schädelknochen in den ersten Wochen nach der Geburt noch weich und formbar sind. Eine Dauerbelastung des Schädels durch die CPAP-Vorrichtung kann zu Druckstellen und Deformationen führen, was jedoch zu vermeiden ist.

Der Erfindung liegt daher die Aufgabe zugrunde, eine CPAP-Vorrichtung bereitzustellen, welche für den Einsatz bei Früh- und Neugeborenen optimiert ist und insbesondere die Belastung auf den Schädel des Früh- und Neugeborenen minimiert.

Die Aufgabe wird erfindungsgemäß gelöst durch eine Vorrichtung zur Erzeugung eines kontinuierlichen positiven Atemwegsdrucks, insbesondere zur Atmungsunterstützung bei Früh- und Neugeborenen, umfassend:
einen eingangsseitigen Einströmkanal, zur Verbindung mit einer Druckluftzufuhr,
einen ausgangsseitigen Sammelkanal, zur Verbindung mit einer Beatmungsmaske oder einem mononasalen Rachentubus oder einem binasalen Prong,
einem Verbindungsstück zwischen eingangsseitigem Einströmkanal und ausgangsseitigem Sammelkanal, wobei das Verbindungsstück wenigstens drei Öffnungen zur Umgebung aufweist,
wobei das Verbindungsstück als Hohlzylinder mit offenen Enden ausgebildet ist und der eingangsseitige Einströmkanal und der ausgangsseitige Sammelkanal gegenüberliegend in der Hohlzylinderwandung des Verbindungsstücks und axial zueinander angeordnet sind,

wobei die Vorrichtung am ausgangsseitigen Sammelkanal einen Verbinder umfasst, zur Verbindung mit einer Beatmungsmaske, einem mononasalen Rachentubus oder einem binasalen Prong,
dadurch gekennzeichnet, dass
die Vorrichtung einstückig ausgebildet ist und der ausgangsseitige Sammelkanal an der Wandung des Verbindungsstücks endet.

Über den eingangsseitigen Einströmkanal wird ein Druckluft-/Sauerstoffstrom in die erfindungsgemäße Vorrichtung eingeleitet, wobei der eingeleitete Druckluft-/ Sauerstoffstrom im Vergleich zur Umgebungsatmosphäre einen erhöhten Druck aufweist. Durch den Innendurchmesser des Einströmkanals wird der Druckluft-/ Sauerstoffstrom kondensiert. Die dadurch erhöhte Flussgeschwindigkeit (Venturi-Effekt) führt beim Austritt der Luft aus dem Einströmkanal in das Verbindungsstück und beim Übergang in den axial angeordneten Sammelkanal zu einem Druckabfall (Bernoulli-Effekt), der bei hohen Luftströmen auch Umgebungsluft anziehen kann. Somit unterstützt die erfindungsgemäße Vorrichtung die Sauerstoffzufuhr bei der Inspiration und sorgt wie beim Stand der Technik für einen verbesserten Gastausch aufgrund des erzeugten Überdrucks.

Das Verbindungsstück weist wenigstens drei, vorzugsweise vier, Öffnungen zur Umgebung auf. Die Öffnungen dienen zum Austritt der Atemluft bei der Exspiration. Die offenen Enden des hohlzylinderförmigen Verbindungsstücks bilden zwei der drei Öffnungen und die dritte Öffnung ist zweckmäßigerweise in der Hohlzylinderwandung des Verbindungsstücks angeordnet. Dadurch wird sichergestellt, dass der Austritt der Atemluft immer möglich ist und ein versehentliches Verschließen aller drei Öffnungen des Verbindungsstücks durch einen Anwender mit nur einer Hand nahezu ausgeschlossen werden kann. Diese Sicherheitsfunktion vermeidet, dass die Lunge des Früh- und Neugeborenen durch einen zu hohen Druck überdehnt wird, respektive Alveolen platzen (Pneumothorax).

Die erfindungsgemäße Vorrichtung weist ein minimalistisches Design mit lediglich einem eingangsseitigen Einströmkanal, einem ausgangsseitigen Sammelkanal und einem Verbindungsstück zwischen den beiden Kanälen auf. Das hohlzylinderförmige Verbindungsstück bildet durch die beiden offen Enden automatisch zwei der drei Öffnungen aus. Dadurch kann die Vorrichtung besonders kompakt und leicht ausgebildet werden, so dass Belastungen des Schädels eines Früh- und Neugeborenen deutlich reduziert werden. Ferner wird durch die axiale Ausrichtung des eingangsseitigen Einströmkanals und des ausgangsseitigen Sammelkanals die Strömung innerhalb des Verbindungsstücks optimiert, wodurch sich die Gesamtgröße und folglich das Gesamtgewicht der Vorrichtung reduziert.

Ein hohlzylindrisches Verbindungsstück optimiert die Druckluft-/Sauerstoffströmung innerhalb des Verbindungsstücks, sowohl bei der Inspiration als auch der Exspiration. Die gewölbte Innenwandung des hohlzylindrischen Verbindungsstücks lenkt den Druckluft-/Sauerstoffstrom besser vom Strömungskanal in den Sammelkanal. Die gewölbte Innenoberfläche des hohlzylindrischen Verbindungsstücks sorgt dafür, dass der durch den eingangsseitigen Einströmkanal in das Verbindungsstück einströmende Luftstrom in den ausgangseitigen Sammelkanal geleitet wird.

Grundsätzlich lässt sich die erfindungsgemäße Vorrichtung mit den unterschiedlichsten Vorrichtungen zur Atemunterstützung nutzen bzw. verbinden, wobei die genannte Beatmungsmaske, der genannte mononasale Rachentubus bzw. der genannte binasale Prong die üblichsten Vorrichtungen zur Atemunterstützung darstellen.

Im Gegensatz zum Stand der Technik gemäß der WO 99/24101 A1 und der EP 1 897 577 A1 verzichtet die erfindungsgemäße Vorrichtung auf den Hohlkörper, in welchem gemäß dem Stand der Technik der positive Druck erzeugt wird. Dadurch wird das Gewicht der erfindungsgemäßen Vorrichtung im Vergleich zu diesem Stand der Technik deutlich reduziert.

Die Vorrichtung gemäß der EP 0 658 356 A1 wird direkt am Kopf des Patienten fixiert und ist daher nur bedingt für den Einsatz bei Früh- und Neugeborenen geeignet.

In einer vorteilhaften Variante der Erfindung sind der eingangsseitige Einströmkanal und der ausgangsseitige Sammelkanal gerade ausgebildet. Die erfindungsgemäße Vorrichtung weist somit einen axialen Strömungskanal vom Eingang des Einströmkanals über das Verbindungsstück bis zum Ausgang des Sammelkanals auf. Dies ist nicht nur förderlich für das Strömungsverhalten innerhalb der erfindungsgemäßen Vorrichtung, sondern ermöglicht auch eine freiere Positionierung des Anschlussschlauchs am eingangsseitigen Einströmkanal. Im Stand der Technik gemäß der WO 99/24101 A1, EP 1 897 577 A1, EP 0 658 356 A1 und der WO 90/24101 A1 ist der eingangsseitige Druckluftanschluss abgewinkelt zum ausgangsseitigen Beatmungsanschluss, was die Positionierung sowohl der Vorrichtung als auch des eingangsseitig angeschlossenen Schlauchs zur Druckluftzufuhr deutlich einschränkt. Jedoch ist es gerade bei Früh- und Neugeborenen wesentlich, dass die Positionierung der Vorrichtung so erfolgt, dass unnötige Belastungen auf den Kopf des Patienten wirkungsvoll vermieden werden. Die erfindungsgemäße Vorrichtung erleichtert die Positionierung durch die axiale Ausgestaltung, da insbesondere der eingangsseitig angeschlossene Druckluftschlauch wesentlich freier positioniert werden kann.

Nach einer zweckmäßigen Variante der Erfindung ist der Verbinder am ausgangsseitigen Sammelkanal ein Female M15-Adapter. Der Adapter ist beispielsweise nach der DIN EN ISO 80369-2 ausgebildet. Durch den Adapter lässt sich die erfindungsgemäße Vorrichtung schnell, einfach und sicher mit einer Beatmungsmaske oder einem mononasalen Rachentubus oder einem binasalen Prong verbinden, welche beispielsweise einen entsprechenden Gegenverbinder, wie einen Male M15-Adapter, aufweisen.

Gemäß einer erfindungsgemäßen Variante umfasst der Verbinder auf dessen Außenfläche Rippen als Verstärkung und/oder Rillen als Griffelement. Durch die Verstärkungsrippen wird ein einfaches Verbinden gewährleistet, da sich der Verbinder während der Handhabung nicht verformt, was beispielsweise das Einführen eines M15 Male Adapters in einen M15 Female Adapter erschweren könnte. Die als Griffelement dienenden Rillen verbessern generell die Handhabung der Vorrichtung.

In einer vorteilhaften Variante der Erfindung erstrecken sich die Rippen von dem Verbindungsstück über die Außenfläche des ausgangsseitigen Sammelkanals und der Außenfläche des Verbinders. Somit wird neben dem Verbinder auch die Außenfläche des Sammelkanals stabilisiert und gegen Verformungen geschützt. Ein negativer Einfluss von Verformungen auf die patientenseitige (ausgangsseitige) Strömung im Sammelkanal wird somit verhindert.

Nach der Erfindung ist die Vorrichtung einstückig ausgebildet. Durch die einstückige Ausbildung der erfindungsgemäßen Vorrichtung lassen sich die Strömungseigenschaften optimieren. Insbesondere werden Toträume im Bereich von Verbindungen, wie beispielsweise einer lösbaren Verbindung zwischen ausgangsseitigem Sammelkanal und optionalem Verbinder vermieden. Derartige Toträume können beispielsweise das Abatmen von CO₂-haltiger Atemluft erschweren und sind schwierig mit den üblichen Verfahren zu sterilisieren und begünstigen die Ansiedlung von Keimen während des Einsatzes. Durch die einstückige Ausgestaltung werden derartige Toträume vermieden, bzw. können nicht durch unsichere oder ungenaue Verbindungsstellen entstehen.

Gemäß einer erfindungsgemäßen Variante ist die Vorrichtung aus Kunststoff hergestellt. Dadurch lässt sich die Vorrichtung einfach und kostengünstig herstellen. Ferner kann das Gewicht der Vorrichtung minimiert werden, wodurch sich die Vorrichtung insbesondere zur Atmungsunterstützung bei Früh- und Neugeborenen eignet. Beispielsweise hat eine erfindungsgemäße Vorrichtung inklusive des optionalen Verbinders in einer einstückigen Ausbildung aus Kunststoff ein Gewicht unter 5 g, insbesondere unter 4 g.

In einer zweckmäßigen Variante der Erfindung weist der eingangsseitige Einströmkanal einen kleineren Innendurchmesser auf als der ausgangsseitige Sammelkanal. Durch den kleineren Innendurchmesser des Einströmkanals wird der Druckluft-/Sauerstoffstrom stärker kondensiert. Die dadurch erhöhte Flussgeschwindigkeit (Venturi-Effekt) führt beim Austritt der Luft aus dem Einströmkanal in das Verbindungsstück und beim Übergang in den axial angeordneten größeren Sammelkanal zu einem optimalen Druckabfall (Bernoulli-Effekt), der bei hohen Luftströmen auch Umgebungsluft anziehen kann.

Nach einer zweckmäßigen Variante weist der eingangsseitige Einströmkanal einen Durchmesser von 2 mm bis 4 mm, insbesondere 3 mm auf.

Gemäß einer weiteren erfindungsgemäßen Variante weist der ausgangsseitige Sammelkanal einen Durchmesser von 3 mm bis 5 mm, insbesondere 4 mm auf.

In einer vorteilhaften Variante der Erfindung ragt der eingangsseitige Einströmkanal in das Verbindungsstück hinein. Dadurch wird insbesondere der Übergang von dem eingangsseitigen Einströmkanal zu dem ausgangsseitigen Sammelkanal im Verbindungsstück optimiert. Das in das Verbindungsstück hineinragende Ende des Einströmkanals wird dabei so positioniert, dass ein ausreichender Druckluft-/ Sauerstoffstrom mit einem gegenüber der umgebenden Atmosphäre erhöhten Druck in den ausgangsseitigen Sammelkanal strömt. Das am Verbindungsstück angeordnete Ende des ausgangsseitigen Sammelkanals endet an der gewölbten Wandung des hohlzylinderförmigen Verbindungsstücks, wodurch die gewölbte Wandung des hohlzylinderförmigen Verbindungsstücks den Druckluft-/ Sauerstoffstrom vom Strömungskanal in den Sammelkanal lenkt.

Nach einer zweckmäßigen Variante der Erfindung beträgt der Abstand zwischen dem eingangsseitigen Einströmkanal und dem ausgangsseitigen Sammelkanal im Bereich des Verbindungsstücks zwischen 3,5 mm und 5,5 mm, insbesondere 4,5 mm. Insbesondere sind die Abmessungen des Verbindungsstücks, des eingangsseitigen Einströmkanals und ausgangsseitigen Sammelkanals so ausgebildet, dass ein Positiver Endexpiratorischer Druck (PEEP) von mindestens 3 cm H₂O bis zu 12 cm H₂O erreicht wird, vorzugsweise zwischen 5 cm H₂O und 8 cm H₂O.

Gemäß einer zweckmäßigen Variante der Erfindung bilden die offenen Enden des hohlzylinderförmigen Verbindungsstücks zwei der mindestens drei Öffnungen. Die mindestens verbleibende dritte Öffnung ist dabei in der Hohlzylinderwandung des Verbindungsstücks angeordnet.

In einer vorteilhaften Variante ist die dritte Öffnung mittig zwischen dem eingangsseitigen Einströmkanal und dem ausgangsseitigen Sammelkanal in der Hohlzylinderwandung angeordnet. In dieser Position hat die dritte Öffnung den geringsten Einfluss auf die Luftströmung innerhalb des Verbindungsstücks, da die Entfernung sowohl zum eingangsseitigen Einströmkanal als auch zum ausgangsseitigen Sammelkanal maximal ist.

Nach einer Variante der Erfindung ist die Höhe des hohlzylinderförmigen Verbindungsstücks kleiner als der Durchmesser des hohlzylinderförmigen Verbindungsstücks.

Gemäß einer weiteren erfindungsgemäßen Variante ist die Höhe des hohlzylinderförmigen Verbindungsstücks größer als der Außendurchmesser des eingangsseitigen Einströmkanals. Das kreisförmige Verbindungsstück dient somit gleichzeitig als Anschlag für einen auf den Einströmkanal aufgesetzten Luftzufuhrschlauch und verhindert ein Verschließen der Öffnungen am Verbindungsstück.

Die Aufgabe wird ferner gelöst durch ein System zur Erzeugung eines kontinuierlichen positiven Atemwegsdrucks, insbesondere zur Atmungsunterstützung bei Früh- und Neugeborenen, umfassend:
eine Druckluftzufuhr, welche einen Luftstrom mit einem konstanten Druck bereitstellt;
eine Beatmungsmaske, einen mononasalen Rachentubus oder einen binasalen Prong für das Früh- oder Neugeborene; und
eine erfindungsgemäße Vorrichtung, wobei der eingangsseitige Einströmkanal über einen ersten Luftschlauch mit der Druckluftzufuhr verbunden ist und der ausgangsseitige Sammelkanal über einen zweiten Luftschlauch mit der Beatmungsmaske, dem mononasalen Rachentubus oder dem binasalen Prong verbunden ist.

In einer erfindungsgemäßen Variante stellt die Druckluftzufuhr einen konstanten Ausgangsdruck zwischen 2,2 und 6,6 bar bereit, insbesondere zwischen 3,3 und 5,5 bar.

Nachfolgend wird die Erfindung anhand von in den Figuren dargestellten Ausführungsbeispielen näher erläutert. Es zeigen:
- Fig. 1: eine perspektivische Ansicht einer ersten Ausführungsform einer erfindungsgemäßen Vorrichtung zur Erzeugung eines kontinuierlichen positiven Atemwegdrucks,
- Fig. 2: eine Schnittansicht einer zweiten Ausführungsform einer erfindungsgemäßen Vorrichtung zur Erzeugung eines kontinuierlichen positiven Atemdrucks,
- Fig. 3: eine erste Ansicht der ersten Ausführungsform aus Fig. 1 bei einer beispielhaften falschen Haltung der erfindungsgemäßen Vorrichtung im Bereich des Verbindungsstücks,
- Fig. 4: eine zweite Ansicht der ersten Ausführungsform aus Fig. 1 bei einer beispielhaften falschen Haltung der erfindungsgemäßen Vorrichtung im Bereich des Verbindungsstücks,
- Fig. 5: eine perspektivische Ansicht einer dritten Ausführungsform einer erfindungsgemäßen Vorrichtung in Verbindung mit einem Luftzufuhrschlauch und einem mononasalen Rachentubus,
- Fig. 6: eine perspektivische Ansicht der Vorrichtung aus Fig. 5 während der Atemunterstützung eines Früh- und Neugeborenen,
- Fig. 7: eine perspektivische Ansicht einer vierten Ausführungsform einer erfindungsgemäßen Vorrichtung in Verbindung mit einem Luftzufuhrschlauch und einem mononasalen Rachentubus,
- Fig. 8: eine perspektivische Ansicht der Vorrichtung aus Fig. 7 während der Atemunterstützung eines Früh- und Neugeborenen, und
- Fig. 9: eine Seitenansicht einer fünften Ausführungsform einer erfindungsgemäßen Vorrichtung.

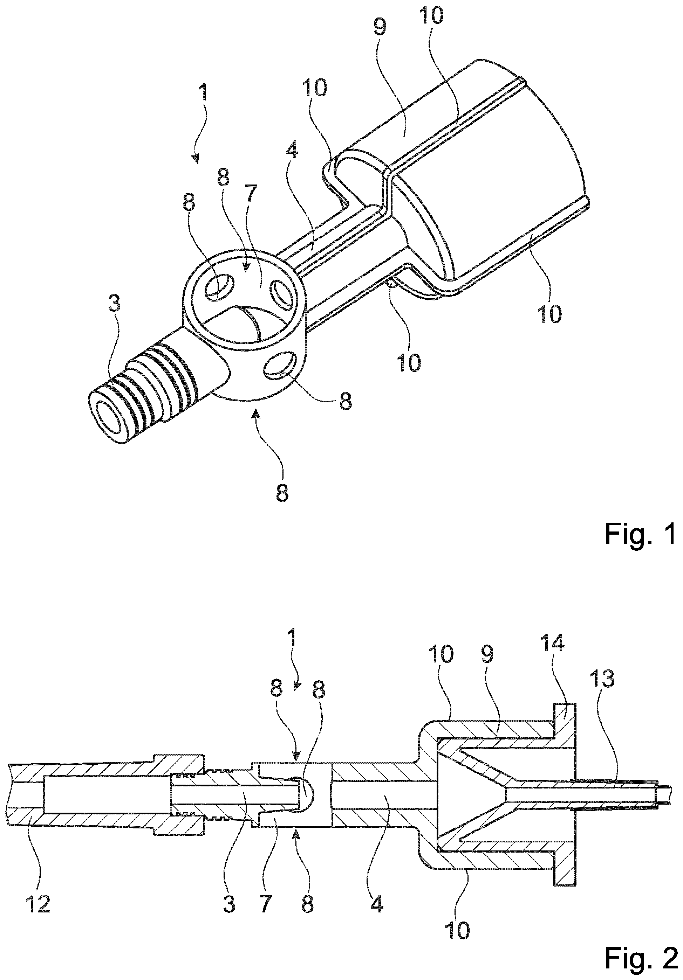

Fig. 1 zeigt eine perspektivische Ansicht einer ersten Ausführungsform einer erfindungsgemäßen Vorrichtung 1 zur Erzeugung eines kontinuierlichen positiven Atemwegdrucks, insbesondere zur Atmungsunterstützung bei Früh- und Neugeborenen 2. Derartige Vorrichtungen sind auch unter der Bezeichnung CPAP (Continuous Positive Airway Pressure) bekannt.

Die Vorrichtung 1 gemäß der in Fig. 1 dargestellten ersten Ausführungsform umfasst einen eingangsseitigen Einströmkanal 3, einen ausgangsseitigen Sammelkanal 4 und ein Verbindungsstück 7 zwischen dem eingangsseitigen Einströmkanal 3 und dem ausgangsseitigen Sammelkanal 4. Ferner umfasst die Vorrichtung 1 einen Verbinder 9 am ausgangsseitigen Sammelkanal 4.

Gemäß der Erfindung ist das Verbindungsstück 7 als Hohlzylinder mit offenen Enden ausgebildet (hohlzylinderförmig). Der eingangsseitige Einströmkanal 3 und der ausgangsseitige Sammelkanal 4 sind gegenüberliegend in der Hohlzylinderwandung des Verbindungsstücks 7 und axial zueinander angeordnet. Ferner sind der eingangsseitige Einströmkanal 3 und der ausgangsseitige Sammelkanal 4 gerade ausgebildet, so dass diese miteinander axial verlaufen.

Das hohlzylinderförmige Verbindungsstück 7 weist wenigstens drei Öffnungen 8 auf, wobei das Verbindungsstück 7 gemäß der ersten Ausführungsform aus Fig. 1 vier Öffnungen 8 aufweist. Über diese mehreren Öffnungen 8 kann die ausgeamtete Luft in die Umgebung entweichen. Die offenen Enden des hohlzylinderförmigen Verbindungsstücks 7 bilden zwei dieser vier Öffnungen 8. Die verbleibenden beiden Öffnungen 8 sind gegenüberliegend auf dem kreisförmigen Verbindungsstück 7 angeordnet und mittig zwischen dem eingangsseitigen Einströmkanal 3 und ausgangseitigen Sammelkanal 4 positioniert. Inklusive der beiden offenen Enden des kreisförmigen Verbindungsstücks 7 sind somit vier Öffnungen 8 in dem Verbindungsstück 7 angeordnet, welche eine Verbindung zur Umgebung herstellen. Dadurch ist es nahezu ausgeschlossen, dass durch eine einzige Hand gleichzeitig alle Öffnungen 8 bedeckt werden können.

Der eingangsseitige Einströmkanal 3 dient zur Verbindung mit einer Druckluftzufuhr (nicht dargestellt), insbesondere einer Druckluft-/Sauerstoffzufuhr. Über den eingangsseitigen Einströmkanal 3 wird von der Druckluftzufuhr ein Druckluft-/ Sauerstoffstrom in die erfindungsgemäße Vorrichtung 1 eingeleitet, wobei der eingeleitete Druckluft-/Sauerstoffstrom im Vergleich zur Umgebungsatmosphäre einen erhöhten Druck aufweist. Beispielsweise ist die Druckluftzufuhr über einen Luftzufuhrschlauch 12 mit dem eingangsseitigen Einströmkanal 3 verbunden, wobei der Luftzufuhrschlauch 12 zweckmäßigerweise auf das freie Ende des eingangsseitigen Einströmkanals 3 aufgesteckt wird.

Durch den Innendurchmesser des Einströmkanals 3 wird der Druckluft-/Sauerstoffstrom kondensiert. Die dadurch erhöhte Flussgeschwindigkeit (Venturi-Effekt) führt beim Austritt der Luft aus dem Einströmkanal 3 in das Verbindungsstück 7 und beim Übergang in den axial angeordneten Sammelkanal 4 zu einem Druckabfall (Bernoulli-Effekt), der bei hohen Luftströmen auch Umgebungsluft anziehen kann. Somit unterstützt die erfindungsgemäße Vorrichtung 1 die Sauerstoffzufuhr bei der Inspiration und sorgt wie beim Stand der Technik für einen verbesserten Gastausch aufgrund des erzeugten Überdrucks. Ferner kann dadurch der Eingangsdruck am Einströmkanal 3 reduziert werden, was die erzeugten Geräusche deutlich reduziert. Eine Geräuschreduzierung ist insbesondere in der Neonatologie vorteilhaft, da die erzeugten Geräusche die Früh- bzw. Neugeborenen 2 zusätzlich stresst.

Der ausgangsseitige Sammelkanal 4 wird mit einer Beatmungsmaske 5 oder einem mononasalen Rachentubus 6 oder einem binasalen Prong verbunden. Dazu ist am ausgangsseitigen Sammelkanal 4 ein Verbinder 9 angeordnet, wie beispielsweise ein Female M15 Adapter gemäß DIN EN ISO 80369-2. Der mononasale Rachentubus 6 ist zweckmäßigerweise direkt oder über einen Verbindungsschlauch 13 mit dem ausgangsseitigen Sammelkanal 4 der erfindungsgemäßen Vorrichtung 1 verbunden, genauso wie beispielsweise eine Beatmungsmaske 5 direkt mit dem Verbinder 9 oder über einen Verbindungsschlauch 13 mit diesem verbunden werden kann. Dazu weist der Verbindungsschlauch 13, die Beatmungsmaske 5, der mononasale Rachentubus 6 oder der binasale Prong beispielsweise ein Verbindungsgegenstück 14, insbesondere einen Male M15 Adapter, auf, welcher in den Female M15 Adapter (Verbinder 9) eingeführt werden kann.

Der Verbinder 9 umfasst auf dessen Außenfläche insgesamt vier Rippen 10 als Verstärkung. Die vier Verstärkungsrippen 10 sind gleichmäßig über den Umfang des Verbinders 9 verteilt, also in einem Abstand von 90°. Gemäß der ersten Ausführungsform aus Fig. 1 erstrecken sich die Verstärkungsrippen 10 von dem Verbindungsstück 7 über die Außenfläche des ausgangsseitigen Sammelkanals 4 und der Außenfläche des Verbinders 9. Somit wird der gesamte ausgangsseitige Bereich der erfindungsgemäßen Vorrichtung 1 verstärkt.

Die Höhe des hohlzylinderförmigen Verbindungsstücks 7 ist gemäß der Ausführungsform aus Fig. 1 größer als der Außendurchmesser des eingangsseitigen Einströmkanals 3. Das kreisförmige Verbindungsstück 7 dient somit gleichzeitig als Anschlag für den Luftzufuhrschlauch 12 und verhindert ein Verschließen der Öffnungen 8 am Verbindungsstück 7.

Die erfindungsgemäße Vorrichtung 1 ist einstückig ausgebildet und insbesondere aus einem Kunststoff hergestellt.

Gemäß der in Fig. 1 dargestellten ersten Ausführungsform weist der eingangsseitige Einströmkanal 3 einen kleineren Durchmesser auf als der ausgangsseitige Sammelkanal 4. Beispielsweise weist der eingangsseitige Einströmkanal 3 einen Durchmesser von 2 mm bis 4 mm, insbesondere 3 mm auf und der ausgangsseitige Sammelkanal 4 einen Durchmesser von 3 mm bis 5 mm, insbesondere 4 mm.

Wie der Fig. 1 weiterhin entnommen werden kann, ragt der eingangsseitige Einströmkanal 3 in das Verbindungsstück 7 hinein und der ausgangsseitige Sammelkanal 4 endet an der Wandung des Verbindungsstücks 7. Der Abstand zwischen dem eingangsseitigen Einströmkanal 3 und dem ausgangsseitigen Sammelkanal 4 im Bereich des Verbindungsstücks 7 beträgt vorzugsweise zwischen 3,5 mm und 5,5 mm, insbesondere 4,5 mm.

Fig. 2 zeigt eine Schnittansicht einer zweiten Ausführungsform einer erfindungsgemäßen Vorrichtung 1 zur Erzeugung eines kontinuierlichen positiven Atemdrucks. Die in Fig. 2 dargestellte zweite Ausführungsform entspricht im Wesentlichen der ersten Ausführungsform aus Fig. 1. In Fig. 2 ist die erfindungsgemäße Vorrichtung 1 am eingangsseitigen Strömungskanal mit einem Luftzufuhrschlauch 12 verbunden, welcher am anderen Ende mit einer Druckluft-/ Sauerstoffzufuhr (Druckluftquelle) verbunden ist. Der Verbinder 9 am ausgangsseitigen Sammelkanal 4 ist über einen entsprechenden Gegenverbinder 14 mit einem Verbindungsschlauch 13 verbunden, welcher wiederum in einen mononasalen Rachentubus 6 übergeht oder mit einem derartigen verbunden ist. Der Fig. 2 lässt sich insbesondere entnehmen, dass der Strömungskanal durch die erfindungsgemäße Vorrichtung 1 axial ist und keine Krümmungen aufweist.

Der Verbinder 9 und der Gegenverbinder 14 sind nicht nur passgenau aufeinander abgestimmt, um eine sichere Verbindung zwischen der erfindungsgemäßen Vorrichtung 1 und dem mononasalen Rachentubus 6, der Beatmungsmaske 5 oder dem binasalen Prong herzustellen, sondern auch, um den Totraum innerhalb der Verbindung zu minimieren. Ein unnötiger vorhandener Totraum erschwert das Abatmen von CO₂, insbesondere für Früh- und Neugeborene 2 und ist daher zu vermeiden oder zumindest zu minimieren.

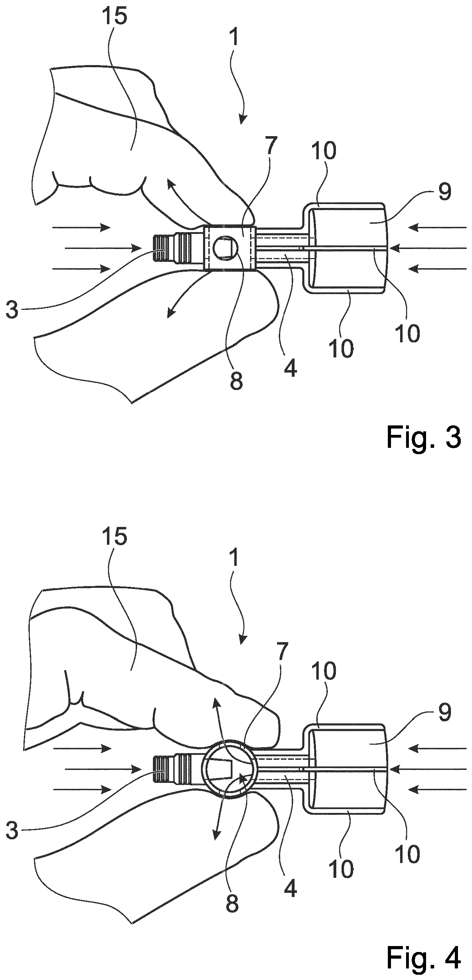

Die Figuren 3 und 4 zeigen eine erste bzw. zweite Ansicht der ersten Ausführungsform aus Fig. 1 bei einer beispielhaften falschen Haltung der erfindungsgemäßen Vorrichtung 1 im Bereich des Verbindungsstücks 7 durch das medizinische Personal. Wie dargestellt, greift die Hand 15 des medizinischen Personals die erfindungsgemäße Vorrichtung 1 im Bereich des Verbindungsstücks 7 und verdeckt dabei teilweise die Öffnungen 8 zur Umgebung. Aufgrund der Anzahl und Anordnung der Öffnungen 8 wird erfindungsgemäß jedoch verhindert, dass das medizinische Personal unabsichtlich gleichzeitig alle Öffnungen 8 zur Umgebung abdeckt und folglich verschließt. Wenn keine Verbindung mehr zur Umgebung besteht, müsste das Früh- und Neugeborene 2 gegen die eingangsseitige Druckluft-/Sauerstoffzufuhr abatmen, was aufgrund der noch nicht voll funktionsfähig ausgebildeten Lungenfunktion nicht möglich ist. Es würde also zu einer Überdehnung der Lunge des Früh- und Neugeborenen kommen, was erfindungsgemäß verhindert wird.

Greift das medizinische Personal die Vorrichtung 1 im Bereich der oberen und unteren Öffnung 8 des hohlzylinderförmigen Verbindungsstücks 7, wie in Fig. 3 dargestellt, kann die abgeatmete Luft über die Öffnungen 8 in der Umfangswandung des Hohlzylinders entweichen. Greift das medizinische Personal die Vorrichtung 1 im Bereich der beiden Öffnungen 8 in der Umfangswandung des hohlzylinderförmigen Verbindungsstücks 7, so kann die abgeatmete Luft aus der oberen und unteren Öffnung 8 des hohlzylinderförmigen Verbindungsstücks 7 entweichen (Fig. 4).

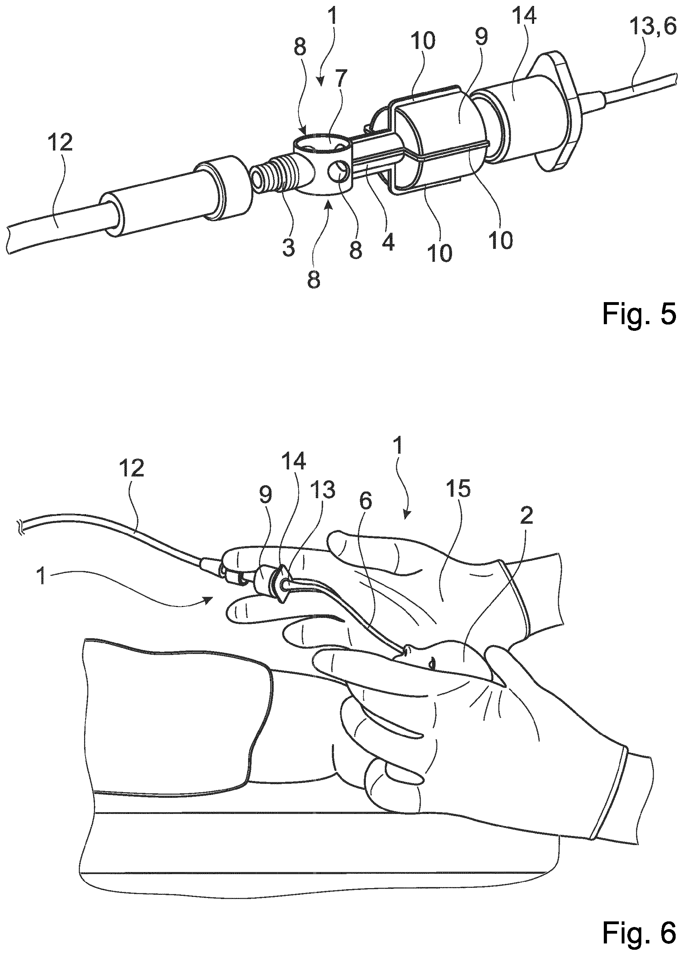

Fig. 5 zeigt eine perspektivische Ansicht einer dritten Ausführungsform einer erfindungsgemäßen Vorrichtung 1 in Verbindung mit einem Luftzufuhrschlauch 12 und einem mononasalen Rachentubus 6. Die Vorrichtung 1 entspricht dabei im Wesentlichen der Vorrichtung aus Fig. 1, welche eingangsseitig mit einem Luftzufuhrschlauch 12 verbunden wird. In den Verbinder 9 am ausgangsseitigen Sammelkanal 4 wird ein Gegenverbinder 14 eingeführt, welcher über einen Verbindungsschlauch 13 mit einem mononasalen Rachentubus 6 verbunden ist oder in diesen übergeht.

Die Kombination aus Verbindungsstück 7 mit der Beatmungsmaske 5, dem mononasalen Rachentubus 6 oder dem binasalen Prong und der verbundenen Druckluftzufuhr (nicht dargestellt) bildet ein erfindungsgemäßes System zur Erzeugung eines kontinuierlichen positiven Atemwegsdrucks, insbesondere zur Atmungsunterstützung bei Früh- und Neugeborenen 2.

Fig. 6 zeigt die Verwendung des Systems bzw. der Vorrichtung 1 aus Fig. 5 während der Atemunterstützung über einen mononasalen Rachentubus eines Früh- und Neugeborenen 2. Der Fig. 6 lässt sich entnehmen, dass die gerade, axiale Ausgestaltung der erfindungsgemäßen Vorrichtung 1 die Führung des Luftzufuhrschlauchs 12 kaum einschränkt, insbesondere im Vergleich zu den aus dem Stand der Technik bekannten abgewinkelten Vorrichtungen.

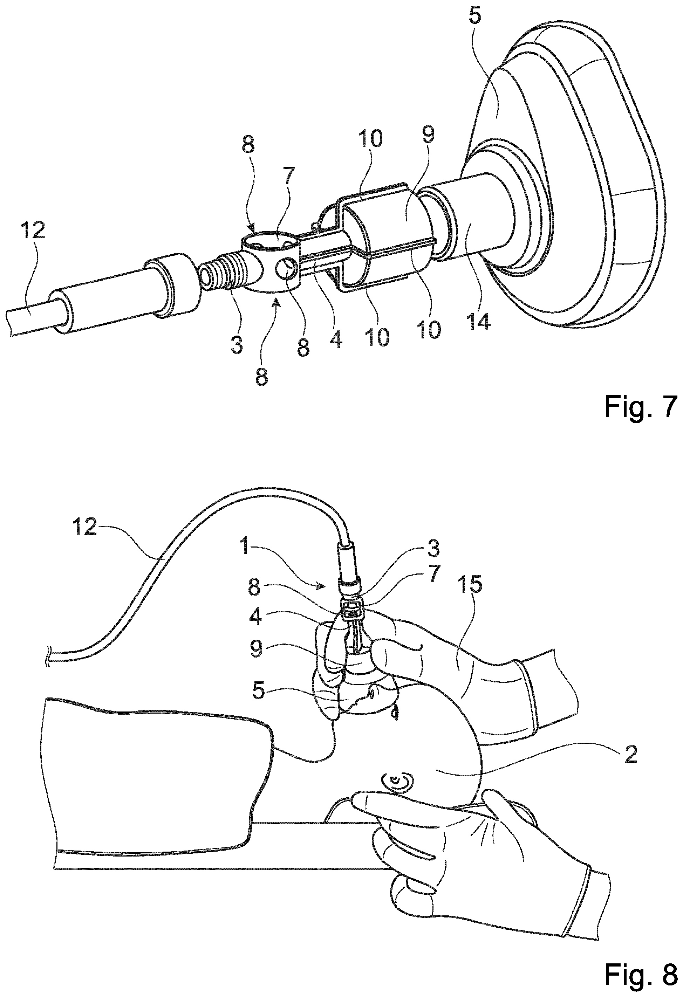

Fig. 7 zeigt eine perspektivische Ansicht einer vierten Ausführungsform einer erfindungsgemäßen Vorrichtung 1 in Verbindung mit einem Luftzufuhrschlauch 12 und einer Beatmungsmaske 5. Die Vorrichtung 1 entspricht dabei im Wesentlichen der Vorrichtung aus Fig. 1, welche eingangsseitig mit einem Luftzufuhrschlauch 12 verbunden wird. In den Verbinder 9 am ausgangsseitigen Sammelkanal 4 wird ein unmittelbar an der Beatmungsmaske 5 angeordneter Gegenverbinder 14 eingeführt.

Fig. 8 zeigt die Verwendung des Systems bzw. der Vorrichtung 1 aus Fig. 7 während der Atmungsunterstützung eines Früh- und Neugeborenen 2 mittels einer Beatmungsmaske 5. Auch hierbei ist ersichtlich, dass die gerade, axiale Ausgestaltung der erfindungsgemäßen Vorrichtung 1 die Führung des Luftzufuhrschlauchs 12 kaum einschränkt.

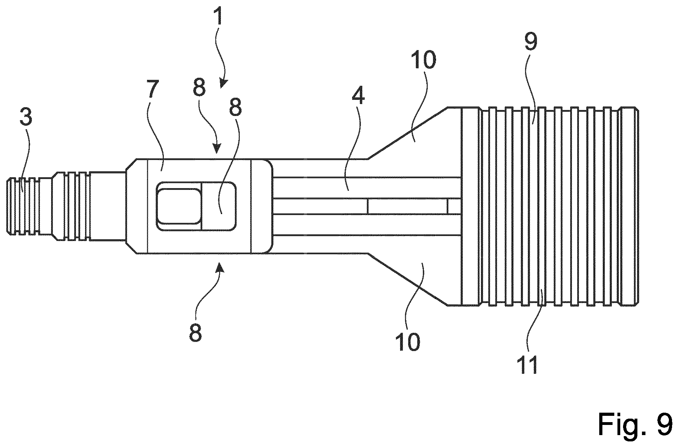

Fig. 9 zeigt eine Seitenansicht einer fünften Ausführungsform einer erfindungsgemäßen Vorrichtung 1 zur Erzeugung eines kontinuierlichen positiven Atemwegsdrucks, insbesondere zur Atmungsunterstützung bei Früh- und Neugeborenen 2. Die fünfte Ausführungsform aus Fig. 9 unterscheidet sich von den vorherigen Ausführungsformen insbesondere dadurch, dass der Verbinder 9 zusätzlich Rillen 11 zur Verbesserung der Griffigkeit aufweist. Die Rillen 11 sind auf der kreisrunden Außenoberfläche des Verbinders 9 angeordnet. Die Verstärkungsrippen 10 erstrecken sich gemäß dem fünften Ausführungsbeispiel von dem Verbindungsstück 7 über den ausgangsseitigen Sammelkanal 4 und entlang der radialen Seitenfläche des Verbinders 9.

Ferner wurde in dem fünften Ausführungsbeispiel die Form der Öffnungen 8 in der kreisrunden Umfangswandung des Verbindungsstücks 7 angepasst, welche nunmehr oval/rechteckig ausgebildet sind und nicht mehr rund wie in den vorherigen dargestellten Ausführungsformen. Grundsätzlich können die Öffnungen 8 jegliche Form aufweisen, solange sie dazu geeignet sind, die abgeatmete Luft aus der Vorrichtung 1 abzuführen.

Die vorliegende Erfindung wird durch die folgenden Ansprüche definiert.

### Bezugszeichenliste

- 1: Vorrichtung
- 2: Früh-/ Neugeborenes
- 3: Einströmkanal
- 4: Sammelkanal
- 5: Beatmungsmaske
- 6: mononasaler Rachentubus
- 7: Verbindungsstück
- 8: Öffnung (Verbindungsstück)
- 9: Verbinder
- 10: Verstärkungsrippe
- 11: Rille (Griffelement)
- 12: Luftzufuhrschlauch
- 13: Verbindungsschlauch (Beatmungsmaske/mononasaler Rachentubus)
- 14: Verbindungsgegenstück
- 15: Hand (medizinisches Personal)

## Patentansprüche

1. Vorrichtung (1) zur Erzeugung eines kontinuierlichen positiven Atemwegsdrucks, insbesondere zur Atmungsunterstützung bei Früh- und Neugeborenen (2), umfassend:
einen eingangsseitigen Einströmkanal (3), zur Verbindung mit einer Druckluftzufuhr,
einen ausgangsseitigen Sammelkanal (4), zur Verbindung mit einer Beatmungsmaske (5) oder einem mononasalen Rachentubus (6) oder einem binasalen Prong,
einem Verbindungsstück (7) zwischen eingangsseitigem Einströmkanal (3) und ausgangsseitigem Sammelkanal (4), wobei das Verbindungsstück (7) wenigstens drei Öffnungen (8) zur Umgebung aufweist,
wobei das Verbindungsstück (7) als Hohlzylinder mit offenen Enden ausgebildet ist und der eingangsseitige Einströmkanal (3) und der ausgangsseitige Sammelkanal (4) gegenüberliegend in der Hohlzylinderwandung des Verbindungsstücks (7) und axial zueinander angeordnet sind,
wobei die Vorrichtung (1) am ausgangsseitigen Sammelkanal (4) einen Verbinder (9) umfasst, zur Verbindung mit einer Beatmungsmaske, einem mononasalen Rachentubus oder einem binasalen Prong,
**dadurch gekennzeichnet, dass**
die Vorrichtung (1) einstückig ausgebildet ist und der ausgangsseitige Sammelkanal (4) an der Wandung des Verbindungsstücks (7) endet.

2. Vorrichtung (1) nach Anspruch 1,
wobei der eingangsseitige Einströmkanal (3) und der ausgangsseitige Sammelkanal (4) gerade ausgebildet sind.

3. Vorrichtung (1) nach Anspruch 1 oder Anspruch 2,
wobei der Verbinder (9) am ausgangsseitigen Sammelkanal (4) ein Female M15-Adapter ist.

4. Vorrichtung (1) nach Anspruch 3,
wobei der Verbinder (9) auf dessen Außenfläche Rippen (10) als Verstärkung und/oder Rillen (11) als Griffelement umfasst.

5. Vorrichtung (1) nach Anspruch 4,
wobei sich die Rippen (10) von dem Verbindungsstück (7) über die Außenfläche des ausgangsseitigen Sammelkanals (4) und der Außenfläche des Verbinders (9) erstrecken.

6. Vorrichtung (1) nach einem der Ansprüche 1 bis 5,
wobei die Vorrichtung (1) aus einem Kunststoff hergestellt ist.

7. Vorrichtung (1) nach einem der Ansprüche 1 bis 6,
wobei der eingangsseitige Einströmkanal (3) einen kleineren Durchmesser aufweist als der ausgangsseitige Sammelkanal (4).

8. Vorrichtung (1) nach einem der Ansprüche 1 bis 7,
wobei der eingangsseitige Einströmkanal (3) in das Verbindungsstück (7) hineinragt.

9. Vorrichtung (1) nach einem der Ansprüche 1 bis 8,
wobei die offenen Enden des hohlzylinderförmigen Verbindungsstücks (7) zwei der drei Öffnungen (8) bilden und die dritte Öffnung (8) in der Hohlzylinderwandung des Verbindungsstücks (7) angeordnet ist.

10. Vorrichtung (1) nach Anspruch 9,
wobei die dritte Öffnung (8) mittig zwischen dem eingangsseitigem Einströmkanal (3) und dem ausgangsseitigem Sammelkanal (4) in der Hohlzylinderwandung angeordnet ist.

11. Vorrichtung (1) nach einem der Ansprüche 1 bis 10,
wobei die Höhe des hohlzylinderförmigen Verbindungsstücks (7) kleiner ist als der Durchmesser des hohlzylinderförmigen Verbindungsstücks (7).

12. Vorrichtung nach einem der Ansprüche 1 bis 11,
wobei die Höhe des hohlzylinderförmigen Verbindungsstücks (7) größer ist als der Außendurchmesser des eingangsseitigen Einströmkanals (3).

13. System zur Erzeugung eines kontinuierlichen positiven Atemwegsdrucks, insbesondere zur Atmungsunterstützung bei Früh- und Neugeborenen (2), umfassend:
eine Druckluftzufuhr, welche einen Luftstrom mit einem konstanten Druck bereitstellt;
eine Beatmungsmaske (5), einen mononasalen Rachentubus (6) oder einen binasalen Prong für das Früh- oder Neugeborene (2); und
eine Vorrichtung (1) gemäß einem der Ansprüche 1 bis 12, wobei der eingangsseitige Einströmkanal (3) über einen ersten Luftschlauch mit der Druckluftzufuhr verbunden ist und der ausgangsseitige Sammelkanal (4) über einen zweiten Luftschlauch mit der Beatmungsmaske (5), dem mononasalen Rachentubus (6) oder dem binasalen Prong verbunden ist.

14. System nach Anspruch 13,
wobei die Druckluftzufuhr einen konstanten Ausgangsdruck zwischen 2,2 und 6,6 bar bereitstellt, insbesondere zwischen 3,3 und 5,5 bar.

## Claims

1. Device (1) for generating a continuous positive airway pressure, in particular for respiratory support in premature and newborn infants (2), comprising:
an inlet channel (3) on the inlet side, for connection to a compressed air supply,
a collecting channel (4) on the outlet side, for connection to a respiratory mask (5) or a mononasal pharyngeal tube (6) or a binasal prong,
a connecting piece (7) between the inlet channel (3) on the inlet side and the collecting channel (4) on the outlet side, the connecting piece (7) having at least three openings (8) to the environment,
the connecting piece (7) is designed as a hollow cylinder with open ends and the inlet channel (3) on the inlet side and the collecting channel (4) on the outlet side are arranged opposite one another in the hollow cylinder wall of the connecting piece (7) and axially to one another,
wherein the device (1) at the collecting channel (4) on the outlet side comprises a connector (9), for connecting with a respiratory mask, a mononasal pharyngeal tube or a binasal prong,
**characterized in that**,
the device (1) is formed in one piece and the collecting channel (4) on the outlet side ends at the wall of the connecting piece (7).

2. Device (1) according to claim 1,
wherein the inlet channel (3) on the inlet side and the collecting channel (4) on the outlet side are straight.

3. Device (1) according to claim 1 or claim 2,
Wherein the connector (9) at the collecting channel (4) on the outlet side, is a female M15 adapter.

4. Device (1) according to claim 3,
wherein the connector (9) comprises on its outer surface ribs (10) as reinforcement and/or grooves (11) as grip element.

5. Device (1) according to claim 4,
wherein the ribs (10) extend from the connector (7) over the outer surface of the collecting channel (4) on the outlet side and the outer surface of the connector (9).

6. Device (1) according to one of claims 1 to 5,
wherein the device (1) is made of a plastic.

7. Device (1) according to one of claims 1 to 6,
wherein the inlet channel (3) on the inlet side has a smaller diameter than the collecting channel (4) on the outlet side.

8. Device (1) according to one of the claims 1 to 7,
wherein the inlet channel (3) on the inlet side projects into the connecting piece (7).

9. Device (1) according to one of the claims 1 to 8
wherein the open ends of the hollow cylindrical connector (7) form two of the three openings (8) and the third opening (8) is arranged in the hollow cylindrical wall of the connector (7).

10. Device (1) according to claim 9,
wherein the third opening (8) is arranged centrally between the inlet channel (3) on the inlet side and the collecting channel (4) on the outlet side in the hollow cylinder wall.

11. Device (1) according to one of claims 1 to 10,
wherein the height of the hollow cylindrical connector (7) is smaller than the diameter of the hollow cylindrical connector (7).

12. Device according to any of the claims 1 to 11,
wherein the height of the hollow cylindrical connecting piece (7) is greater than the outer diameter of the inlet channel (3) on the inlet side.

13. A system for providing continuous positive airway pressure, particularly for respiratory support in premature and newborn infants (2), comprising:
a compressed air supply providing a flow of air at a constant pressure;
a respiratory mask (5), a mononasal pharyngeal tube (6) or a binasal prong for the premature or newborn infant (2); and
a device (1) according to one of claims 1 to 12, wherein the inlet channel (3) on the inlet side is connected to the compressed air supply via a first air hose and the collection channel (4) on the outlet side is connected to the respiratory mask (5), the mononasal pharyngeal tube (6) or the binasal prong via a second air hose.

14. System according to claim 13,
wherein the compressed air supply provides a constant output pressure between 2.2 and 6.6 bar, in particular between 3.3 and 5.5 bar.

## Revendications

1. Dispositif (1) de génération d'une pression positive continue des voies aériennes, en particulier pour un support respiratoire chez des nourrissons prématurés et nouveau-nés (2), comprenant :
un canal d'entrée (3) sur le côté d'entrée, pour le raccordement à une alimentation en air comprimé,
un canal de collecte (4) sur le côté de sortie, pour le raccordement à un masque respiratoire (5) ou à un tube pharyngien mononasal (6) ou à une canule binasale,
une pièce de raccordement (7) entre le canal d'entrée (3) sur le côté d'entrée et le canal de collecte (4) sur le côté de sortie, la pièce de raccordement (7) ayant au moins trois ouvertures (8) sur l'environnement,
la pièce de raccordement (7) est conçue sous la forme d'un cylindre creux à extrémités ouvertes et le canal d'entrée (3) sur le côté d'entrée ainsi que le canal de collecte (4) sur le côté de sortie sont agencés de manière opposée l'un à l'autre dans la paroi du cylindre creux de la pièce de raccordement (7) et axialement l'un par rapport à l'autre,
dans lequel le dispositif (1) au niveau du canal de collecte (4) sur le côté de sortie comprend un connecteur (9), pour se raccorder à un masque respiratoire, à un tube pharyngien mononasal ou à une canule binasale,
**caractérisé en ce que**,
le dispositif (1) est formé d'un seul tenant et le canal de collecte (4) au niveau du côté de sortie termine au niveau de la paroi de la pièce de raccordement (7).

2. Dispositif (1) selon la revendication 1,
dans lequel le canal d'entrée (3) sur le côté d'entrée et le canal de collecte (4) sur le côté de sortie sont droits.

3. Dispositif (1) selon la revendication 1 ou la revendication 2,
dans lequel le connecteur (9) au niveau du canal de collecte (4) sur le côté de sortie est un adaptateur femelle M15.

4. Dispositif (1) selon la revendication 3,
dans lequel le connecteur (9) comprend, sur sa surface externe, des nervures (10) correspondant à des renforts et/ou des rainures (11) servant d'éléments de préhension.

5. Dispositif (1) selon la revendication 4,
dans lequel les nervures (10) s'étendent à partir du connecteur (7) sur la surface externe du canal de collecte (4) au niveau du côté de sortie et sur la surface externe du connecteur (9).

6. Dispositif (1) selon l'une des revendications 1 à 5,
dans lequel le dispositif (1) est fabriqué en plastique.

7. Dispositif (1) selon l'une des revendications 1 à 6,
dans lequel le canal d'entrée (3) sur le côté d'entrée possède un diamètre inférieur à celui du canal de collecte (4) sur le côté de sortie.

8. Dispositif (1) selon l'une des revendications 1 à 7,
dans lequel le canal d'entrée (3) sur le côté d'entrée fait saillie dans la pièce de raccordement (7).

9. Dispositif (1) selon l'une des revendications 1 à 8,
dans lequel les extrémités ouvertes du connecteur cylindrique creux (7) forment deux des trois ouvertures (8) et la troisième ouverture (8) est agencée dans la paroi cylindrique creuse du connecteur (7).

10. Dispositif (1) selon la revendication 9,
dans lequel la troisième ouverture (8) est agencée de manière centrale entre le canal d'entrée (3) sur le côté d'entrée et le canal de collecte (4) sur le côté de sortie dans la paroi du cylindre creux.

11. Dispositif (1) selon l'une des revendications 1 à 10,
dans lequel la hauteur du connecteur cylindrique creux (7) est inférieure au diamètre du connecteur cylindrique creux (7).

12. Dispositif selon l'une quelconque des revendications 1 à 11,
dans lequel la hauteur de la pièce de raccordement cylindrique creuse (7) est supérieure au diamètre externe du canal d'entrée (3) sur le côté d'entrée.

13. Un système de fourniture d'une pression positive continue des voies aériennes, en particulier pour un support respiratoire chez des nourrissons prématurés et nouveau-nés (2), comprenant :
une alimentation en air comprimé fournissant un flux d'air à une pression constante ;
un masque respiratoire (5), un tube pharyngien mononasal (6) ou une canule binasale pour le nourrisson prématuré ou nouveau-né (2) ; et
un dispositif (1) selon l'une des revendications 1 à 12, dans lequel le canal d'entrée (3) sur le côté d'entrée est raccordé à l'alimentation en air comprimé par l'intermédiaire d'un premier tuyau d'air et le canal de collecte (4) sur le côté de sortie est raccordé au masque respiratoire (5), au tube pharyngien mononasal (6) ou à la canule binasale par l'intermédiaire d'un deuxième tuyau d'air.

14. Système selon la revendication 13,
dans lequel l'alimentation en air comprimé fournit une pression de sortie constante comprise entre 2,2 et 6,6 bars, en particulier entre 3,3 et 5,5 bars.
